# EUROPEAN PATENT APPLICATION

(11) **EP 4 353 367 A1**
(43) Date of publication of application: **17.04.2024**
(21) Application number: 23199272.8
(22) Date of filing: 25.09.2023
(51) Int. Cl.: B05C 17/005

(54) **COMPULE AND SYRINGE TIP HAVING DISCHARGE NOZZLE DIAMETER WIDENED AT TIP**

(30) Priority: 26.09.2022 JP 2022152909
(71) Applicant: Shofu Inc., Kyoto-shi, Kyoto 605-0983 (JP)
(72) Inventor: SHIGA, Toshio, Tokyo, 103-8538 (JP); SASAKI, Tsukasa, Tokyo, 103-8538 (JP); TAKEI, Ryoji, Tokyo, 103-8538 (JP); NAKATSUKA, Toshiyuki, Kyoto, 605-0983 (JP); MIYATA, Shunsuke, Kyoto, 605-0983 (JP); KOBAYASHI, Hiroyuki, Kyoto, 605-0983 (JP); MATSUMOTO, Akiko, Kyoto, 605-0983 (JP)
(74) Representative: Eisenführ Speiser

(57) **Abstract**

To provide a compule or syringe tip that can disharge viscous material in an intended shape by reducing deformation of the viscous material during discharge, can prevent viscous material from adhering to unintended portion by reducing an amount of viscous material that adheres to a nozzle tip, and can reduce dripping of viscous material to improve the anti-dripping property of the viscous material.

To provide a compule or syringe tip having a discharge structure for discharging viscous material from a discharge opening,wherein the discharge structure comprises a flow path that is formed around a virtual central line and allows the viscous material to move, and a tapered flow path formed between the flow path and the discharge opening, wherein a cross-sectional area of the tapered flow path gradually increases toward the discharge opening.

## Description

### BACKGROUND OF THE INVENTION

### Field of the Invention

The present invention relates to a compule or a syringe tip having a discharge structure that discharges viscous material from a discharge opening.

### Description of the Related Art

In the dental field, pastes such as dental filling materials and materials for preparing dental crowns may be filled into a compule as described in US Pat. No. 4,391,590 or an injector such as a syringe as described in US Pat. No. 5,445,523 in order to optimize a discharge amount and used.

### SUMMARY OF THE INVENTION

### Technical Problem

In the conventional compule described in US Pat. No. 4,391,590 and the conventional syringe tip described in US Pat. No. 5,445,523, due to the characteristics of the paste (viscous material), there is a case that a part of the paste discharged from the discharge opening adheres to an annular tip surface surrounding the discharge opening and an outer wall portion of the tip of the ijector, such as the outer peripheral wall along the flow path of the ijector via this tip surface. When the amount of the viscous material attached to the outer wall portion of the tip of the ijector becomes large, it falls from the outer wall portion of the tip of the syringe, and therefore the viscous material adheres to areas that are not intended for application.

Therefore, in the conventional injectors, there has been a problem in that it is difficult to apply the viscous material to a desired portion. Also, if the viscous material attached to the outer wall portion of the tip of the ijector falls to the desired position, more viscous material than the desired amount is applied. Therefore, there has been a problem in that it is difficult to apply the desired amount of viscous material. Furthermore, if the viscous material adhering to the outer wall portion of the tip of the compule does not fall off, the adhering viscous material will be discarded, and therefore there has been a problem in that usage efficiency of the viscous material is low.

An object of the present invention is to provide a compule or syringe tip that can disharge viscous material in an intended shape by reducing deformation of the viscous material during discharge, can prevent viscous material from adhering to unintended portion by reducing an amount of viscous material that adheres to a nozzle tip, and can reduce dripping of viscous material to improve the anti-dripping property of the viscous material.

Another object of the present invention is to provide a compule or syringe tip that can reduce an amount of viscous material adhering to an outer wall portion, preferably prevents adhesion of viscous material to an outer wall portion to apply a desired amount of viscous material to a desired portion to increase the efficiency of using viscous material.

### Solution to Problem

The present invention provides, as one embodiment, a compule or syringe tip having a discharge structure for discharging viscous material from a discharge opening,wherein the discharge structure comprises a flow path that is formed around a virtual central line and allows the viscous material to move, and a tapered flow path formed between the flow path and the discharge opening, wherein a cross-sectional area of the tapered flow path gradually increases toward the discharge opening.

### Advantageous Effects of Invention

The present invention can provide a compule or syringe tip that can disharge viscous material in an intended shape by reducing deformation of the viscous material during discharge, can prevent viscous material from adhering to unintended portion by reducing an amount of viscous material that adheres to a nozzle tip, and can reduce dripping of viscous material to improve the anti-dripping property of the viscous material

### Brief description of drawings

[FIG. 1] Schematic view showing state in which compule having disharge structure according to one embodiment is attached to ejector holder.
[FIG. 2] Perspective view of compule according to one embodiment.
[FIG. 3] Left side view of compule according to one embodiment.
[FIG. 4] Cross-sectional view along A-A plane in Figure 2.
[FIG. 5] Enlarged sectional view of discharge opening portion of compule according to one embodiment.
[FIG. 6] Side view of cap of compule according to one embodiment.
[FIG. 7] Sectional view of compule in state of attaching cap according to one embodiment.
[FIG. 8] Perspective view of compule according to another embodiment.
[FIG. 9] Left side view of compule according to another embodiment.
[FIG. 10] Sectional view along B-B line in Figure 8.
[FIG. 11] Enlarged sectional view of discharge opening portion according to another embodiment.
[FIG. 12] Perspective view of piston.
[FIG. 13] Schematic view showing of discharge opening portion of compule according to one embodiment.
[FIG. 14] Schematic view showing configuration of syringe and syringe tip according to one embodiment.
[FIG. 15] Front view of syringe.
[FIG. 16] Plan view of syringe.
[FIG. 17] Sectional view along I-I line in Figure 15.
[FIG. 18] Front view of push rod according to one embodiment.
[FIG. 19] Plan view of push rod according to one embodiment.
[FIG. 20] Sectional view along III-III line in Figure 18.
[FIG. 21] Front view of syringe tip.
[FIG. 22] Plan view of syringe tip.
[FIG. 23] Sectional view along II-II line in Figure 21.
[FIG. 24] Enlarged sectional view of discharge opening portion of syringe tip.
[FIG. 25] Schematic view of discharge opening portion of syringe tip.
[FIG. 26] Sectional view of syringe tip and syringe in state of attaching syringe tip to tip of syringe.

### DETAILED DESCRIPTION OF THE PREFERRED EMBODIMENTS

In one embodiment of the present invention, in the cross section of the flow path including the virtual center line, an angle between an extending direction of a peripheral wall of the flow path in the end portion on a discharge opening side and an extending direction of a peripheral wall of the tapered flow path, may be 5° to 85°.

In one embodiment of the present invention, the tapered flow path may have a truncated conical shape.

In one embodiment of the present invention, the flow path may have a truncated conical shape or a columnar shape.

In one embodiment of the present invention, the tapered flow path and an outer peripheral wall portion of a discharge container or an outer peripheral wall portion of a discharge instrument may be connected by a circular tip surface.

In one embodiment of the present invention, the circular tip surface has an annular shape.

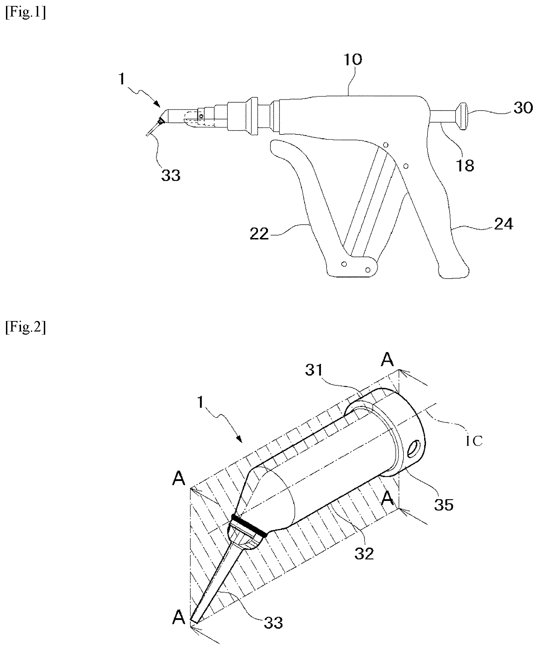

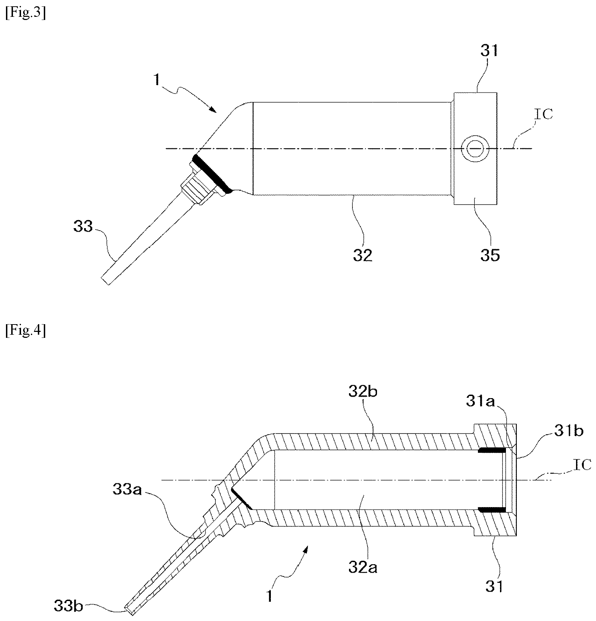

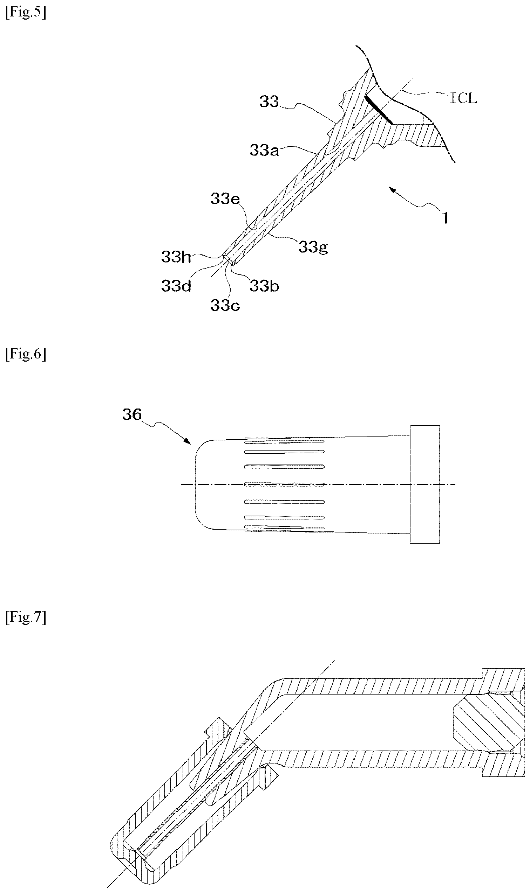

Hereinafter, specific embodiments of the present invention will be described in detail with reference to the drawings. Figure 1 is a schematic view showing a state in which a compule 1 having a disharge structure according to one embodiment of the present invention is attached to an ejector holder 10. Figure 2 is a perspective view of the compule 1. Figure 3 is a left side view of the compule 1. Figure 4 is a sectional view along A-A plane in Figure 2. Figure 5 is an enlarged sectional view of the discharge opening portion.

The ejector holder 10 has a barrel, and the barrel has an inner hole extending from the rear end toward the front end. The inner hole is inserted with a plunger 18 having substantially the same diameter as the inner hole 12, and the front end of the plunger 18 is provided with a extending portion having a small diameter. The rear end of the barrel is fixed with a first handle member 24 extending incliend with respect to the longitudinal direction of the barrel. The first handle member 24 is provided with a second handle member via a connecting member. The rear end of the plunger 18 is provided with a button 30 and by pressing this button 30, the plunger 18 is displaced toward the compule 1 side.

The compule 1 may be formed of, for example, a resin material or a metal material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the compule 1 is made of polypropylene.

The compule 1 of the present embodiment includes an engaging portion 31 that engages with the ejector holder 10, a cylindrical portion 32 that has a hollow portion 32a having the center on the virtual central line IC, and a nozzle portion 33. The hollow portion 32a of the cylindrical portion 32 is filled with a viscous material, and the nozzle portion 33 discharges this viscous material. The viscous material may be a dental material such as a filling composite resin, a sealant material, a cement, a bonding material, a dental splinting material, an etching material, and a tooth surface polishing material.

The engaging portion 31 of the present embodiment is formed with a hollow portion 31a having the center on the central axis. Therefore, the engaging portion 31 of the present embodiment has a cylindrical shape. The inside of the engaging portion 31 is provided with a piston 34. The outer diameter of the engaging portion 31 may be, for example, 2.5 mm to 15 mm, and the dimension in the longitudinal direction of the compule 1 may be, for example, 10 mm to 50 mm. The inner diameter of the engaging portion 31 of the present embodiment may be, for example, 1 mm to 13 mm. One end of the hollow portion 31a constitutes an insertion opening 31b inserted with the plunger 18. The other end of the hollow portion 31a is continuous with the hollow portion 32a of the cylindrical portion 32. The outer peripheral portion of the engaging portion 31 is provided with a circular flange 35.

The cylindrical portion 32 of the present embodiment is an annular member having the center on the virtual central line IC, and has a hollow portion 32a having the center on the virtual central line IC in the inside of the cylindrical portion 32. Specifically, the cylindrical portion 32 of the present embodiment has a main body portion 32b having a cylindrical shape and being formed with a hollow portion 32a in the inside of the main body portion 32b. The outer diameter of the cylindrical portion 32 may be, for example, 2.5 mm to 15 mm, and the dimension in the longitudinal direction of the compule 1 may be, for example, 9 mm to 48 mm. One end of the hollow portion 32a of the present embodiment is connected to the hollow portion 31a of the engaging portion 31 and may have an inner diameter of, for example, 1 mm to 13 mm. The main body portion 32b of the present embodiment is formed so that a cross-sectional size is constant. Further, the hollow portion 32a of the present embodiment is formed so that a cross-sectional size is constant. The other end of the hollow portion 32a of the present embodiment is continuous with the flow path of the viscous material formed in the inside of the nozzle portion 33.

In the present specification, the virtual central line of a hollow portion is defined as a line connecting virtual centers from the portion adjacent to the engaging portion to the portion adjacent to the nozzle in the case that the maximum feret diameter in the cross section of the hollow portion perpendicular to the nozzle direction from the engaging portion is calculated, the maximum Feret diameter is defined as the diameter, and the center of the virtual circle contacting with the cross-sectional outline of the hollow portion is defined as the virtual center.

In the present embodiment, the virtual central line IC positioned at the center of the hollow portion 32a is coaxial with the central axis of the hollow portion 31a.

The nozzle portion 33 discharges the viscous material contained in the hollow portion 32a. Specifically, the nozzle portion 33 of the present embodiment is formed with a flow path 33a of viscous material in the inside of the nozzle portion 33, and one end of the flow path 33a is connected to a discharge opening 33b. A dimension of the nozzle portion 33 in the longitudinal direction of the cylindrical portion 32 may be, for example, 1 mm to 40 mm. Further, the dimension of the nozzle portion 33 in the direction in which the flow path 33a extends may be 5 mm or more, and may be 8 mm or more. By setting the dimension of the nozzle portion in the direction in which the flow path 33a extends to such a length, it is possible to easily fill the molars which are at the innermost part of the oral cavity with the viscous material, for example.

The nozzle portion 33 of the present embodiment is formed in a manner such that the cross-sectional size decreases toward the discharge opening 33b. However, the nozzle portion 33 may be formed in a manner such that the cross-sectional size is constant toward the discharge opening 33b and may be formed in a manner such that the cross-sectional size increases toward the discharge opening 33b. Further, the flow path 33a of the present embodiment is formed into a columnar shape so that the cross-sectional size is constant toward the discharge opening 33b. However, the flow path 33a may be formed in a manner such that the cross-sectional size dereases toward the discharge opening 33b and may be formed in a manner such that the cross-sectional size increases toward the discharge opening 33b.

The nozzle portion 33 of the present embodiment extends in a direction inclining with respect to the virtual central line IC of the hollow porion 32a of the cylindrical portion 32.

The flow path 33a of the present embodiment is formed around the virtual central line ICL, and a tapered flow path 33c whose cross-sectional area gradually increases toward the discharge opening 33b is formed between the flow path 33a and the discharge opening 33b. In the present embodiment, the flow path 33a and the tapered flow path 33c are formed in the nozzle portion 33, and therefore, in the present embodiment, the discharge structure of the present invention is configured in the nozzle portion 33.

The tapered flow path 33c of the present embodiment has a truncated conical shape. Therefore, in the cross section including the virtual central line of the flow path 33a, the peripheral wall 33d of the tapered flow path 33c has a linear shape.

As shown in Fig. 5, the flow path 33a of the present embodiment is formed around the virtual central line ICL. In the present specification, the virtual central line of the flow path may be defined as a line connecting the centers of gravity of the flow path in a cross section perpendicular to the moving direction of the viscous material. The nozzle portion 33 of the present embodiment has a tapered flow path 33c that is formed between the flow path 33a and the discharge opening 33b and the cross-sectional area of the tapered flow path 33c gradually increases toward the discharge opening 33b.

In the nozzle portion 33 of the present embodiment, a cross-sectional area gradually increases toward the discharge opening 33b in a portion of the tapered flow path that is continuous with the discharge opening 33b. Therefore, it is possible to decrease the internal pressure of the viscous material flowing from the flow path 33a toward the discharge opening 33b in the tapered portion 33c, thereby reducing inherent strain. If the strain is present in the discharged viscous material, internal pressure is released during discharge, and deformation of the viscous material is caused. By reducing the strain, it is possible to reduce the deformation of the viscous material during discharge and to discharge the viscous material in the intended shape.

Further, in the nozzle portion 33 of the present embodiment, there is the tapered portion 33c that is continuous with the discharge opening 33b. Because the tapered portion 33c presents, the area of the circular tip surface 33h decreases, therefore the amount of viscous material that adheres to the circular tip surface 33h inevitably decrease. Thus, it is prevented the inner surface of the cap from becoming dirty when the cap is fitted. In addition, the adhesion of the viscous material to undesired portion due to dripping of the paste adhering to the circular tip surface 33h is also suppressed. Furthermore, because the tapered portion 33c presents, it is possible that the viscous material discharged through the peripheral wall 33e, the peripheral wall 33d and the circular tip surface 33h pass smoothly without being caught at the edge of the discharge opening. Therefore, when the paste is pulled back by returning to the original position in the case of stopping the discharge of the viscous material, the viscous material is pulled back into the nozzle without remaining on the circular tip surface 33h. Thus, dripping of the viscous material can be reduced. Furthermore, the viscous material is smoothly pulled back into the nozzle, it is possible to improve the anti-dripping property of the viscous material.

Furthermore, in the nozzle portion 33 of the present embodiment, the cross-sectional area gradually increases toward the discharge opening 33b in the tapered portion 33c that is continuous with the discharge opening 33b, and therefore the internal volume of the nozzle portion increases. Thus, even if the pull back of the paste occurs by pull back of the plunger 30, because of increasing the paste capacity within the nozzle, it is possible that the air sucked from the tip portion remains in the nozzle tip to reduce the inclusion of air bubbles.

The tapered flow path 33c of the present embodiment has a truncated conical shape. Therefore, in the cross section including the virtual central line of the flow path 33a, the peripheral wall 33d of the tapered flow path 33c has a linear shape. In this way, by forming the peripheral wall 33d of the tapered flow path 33c into a linear shape in the cross section including the virtual central line of the flow path 33a, it is possible to improve the anti-dripping property of the viscous material.

In the nozzle portion 33 of the present embodiment, in the cross section of the flow path 33a including the virtual central line ICL of the flow path 33a, it is preferable that the angle between the extending direction of the peripheral wall 33e of the flow path 33a in the end portion on the discharge opening 33b side and the extending direction of the peripheral wall 33d of the tapered flow path 33c is 5° to 85°, more preferably 10° to 60°. By setting the angle to 5° to 85°, it is possible to prevent the viscous material from adhering to the outer wall portion 33f of the nozzle portion 33. In the present embodiment, the angle between the extending direction of the peripheral wall 33e of the flow path 33a in the end portion on the discharge opening 33b side and the extending direction of the peripheral wall 33d of the tapered flow path 33c is 45°.

In the nozzle portion 33 of the present embodiment, the tapered flow path 33c and the outer peripheral wall portion 33g of the nozzle portion 33 are connected by a circular tip surface 33h. In particular, in the present embodiment, since the circular tip surface 33h has a flat annular shape, it is possible to improve the anti-dripping property of the viscous material.

Figure 6 is a side view of the cap 36 of the compule according to one embodiment, and Figure 7 is a sectional view of the compule, the cap, and the piston according to the embodiment in the state of attaching the cap 36. The compule 1 of the present embodiment is configured in a manner such that storage stability is improved by improving the light-shielding properties of the nozzle portion and unintended dripping of the viscous material is prevent, by attaching the cap 36 from the nozzle portion side in storing or in discontinuing use.

In the compule of the above embodiment, the nozzle portion, the cylindrical portion and the engaging portion are integrally formed from a resin material. Therefore, press-fitting of the nozzle is not required during manufacturing, and it is possible to facilitate manufacturing. Further, since the nozzle portion, the cylindrical portion and the engaging portion are made of only resin material, there is no need to separate metals at the time of disposal, and disposal can be easily performed.

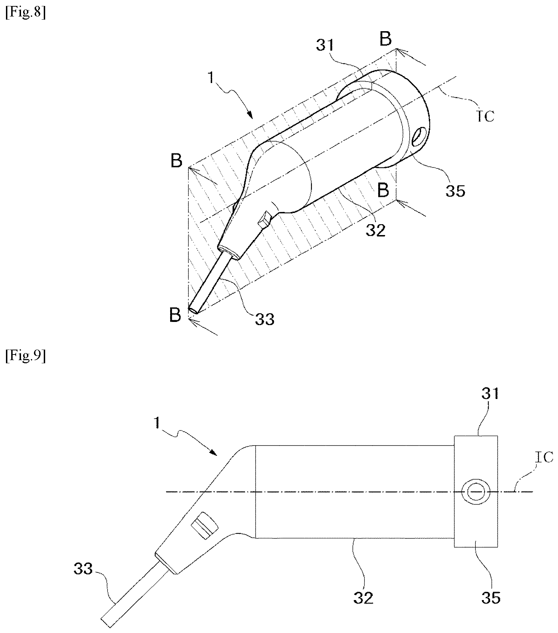

In the compule of the above embodiment, the nozzle portion, the cylindrical portion and the engaging portion are integrally formed, however, the nozzle portion, the cylindrical portion and the engaging portion may be individually formed as separate members, as the compule showin in Figures 8 to 11.

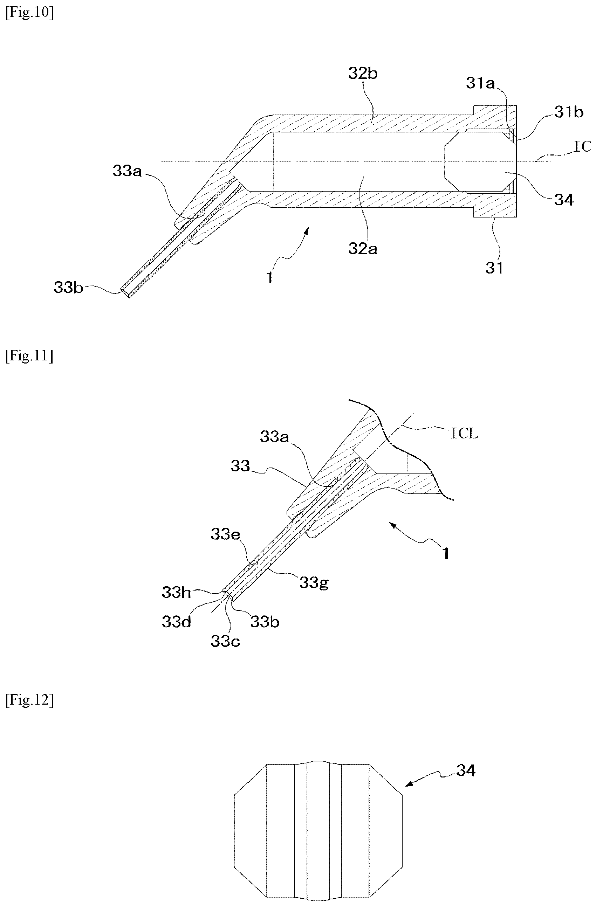

As shown in Figure 12, the piston 34 of the present embodiment is configured to have a substantially columnar shape.

As the viscous material in the above embodiment, dental materials, pharmaceuticals, medical materials, cosmetics, foods and the like may be used.

In the above embodiments, the compule has a symmetrical shape, but the compule may have an asymmetrical shape.

The compule of the above embodiment is configured in a manner such that an extending direction of the nozzle portion is bent from an extending direction of the cylindrical portion, however, the nozzle portion and the cylindrical portion may be configured linearly.

In the compule of the above embodiment, the flow path of the nozzle portion is formed linearly, however, the flow path of the nozzle portion may be formed in a curved shape.

In the compule of the above embodiment, the nozzle is formed in a manner such that the cross-sectional size is constant toward the discharge opening, however, the cross-sectional size of the nozzle portion may decrease toward the discharge opening.

In the compule of the above embodiment, the flow path is formed in a manner such that the cross-sectional size is constant toward the discharge opening, however, the cross-sectional size of the flow path may decrease toward the discharge opening.

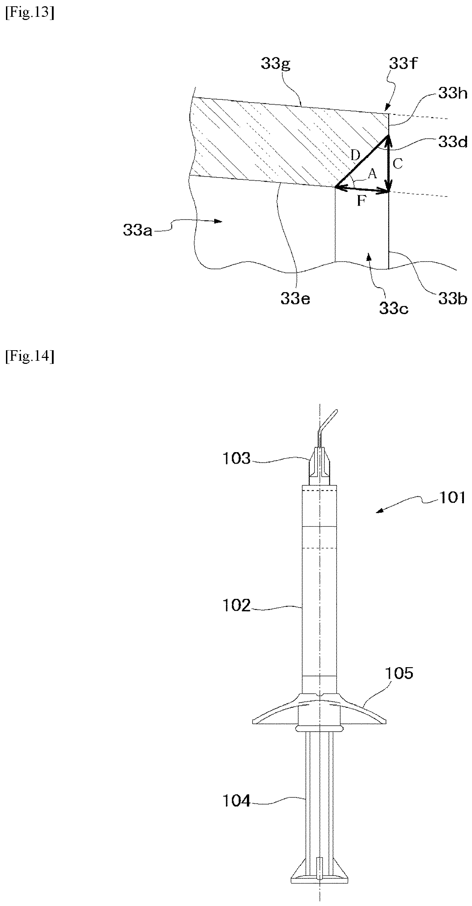

Figure 14 is a schematic view showing a configuration of a nozzle exchangeable injector 101 having a discharging structure according to one embodiment of the present invention. In the present embodiment, the injector 101 comprises a syringe 102 and a syringe tip 103 that is detachably and threadably attached to a tip 121 of the syringe 102. The syringe tip 103 of the present embodiment is attached and fixed to the tip porion of the syringe 102 by rotating operation in a predetermined direction from the tip of the syringe 102.

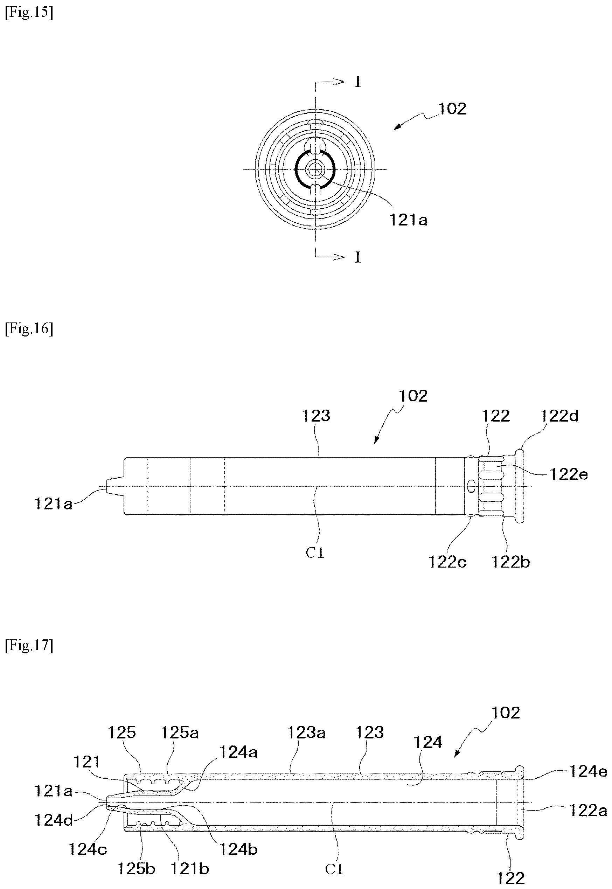

Figure 15 is a front view of syringe 102, Figure 16 is a plan view of syringe 102 and Figure 17 is a sectional view along I-I line in Figure. The syringe 102 is also sometimes referred to as a barrel. The syringe 102 is an annular member having the center on the central axis C1, and is made of, for example, a resin material or a glass material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the syringe 102 is consisting of polypropylene.

The syringe 102 of the present embodiment comprises a push rod 104. The push rod 104 is also called a plunger. The syringe 102 of the present embodiment comprises a tip portion 121 having a discharge opening 121a at one end, a rear end portion 122 having at one end an insertion opening 122a inserted with the push rod 104 described later, and the main body portion 123 positioned between the tip portion 121 and the rear end portion 122. The outer diameter of the syringe 102 may be, for example, 3 mm to 15 mm.

The tip portion 121 of the present embodiment is formed into a tapered cylindrical shape so that a cross-sectional size decreases toward the discharge opening 121a. The dimension of the tip portion 121 in the longitudinal direction of the syringe may be, for example, 1 mm to 20 mm. The main body portion 123 of the present embodiment is formed into a cylindrical shape so that a cross-sectional size is constant. The dimension of the main body portion 123 in the longitudinal direction of the syringe may be, for example, 30 mm to 150 mm. The rear end portion 122 of the present embodiment is formed with a finger grip attachment portion 122b to which a finger grip 105 is attached. The dimension of the rear end portion 122 in the longitudinal direction of the syringe may be, for example, 1 mm to 20 mm.

The finger grip attachment portion 122b of the present embodiment includes a pair of flange portions 122c and 122d formed over the entire circumferential surface of the syringe 102, and eight recess portions 122e formed between the pair of flange portions 122c and 122d. The pair of flange portions 122c and 122d restricts movement of the finger grip 105 along the longitudinal direction of the syringe 102. In the present embodiment, the flange portion 122d is formed larger than the flange portion 122c. Further, in the present embodiment, the flange portion 122d is formed at the rear end of the rear end portion 122. The eight recess portions 122e are fitted with eight protrusions (not shown) provided in the hollow portion (not shown) of the finger grip 105. Due to this fitting, rotation of the finger grip 105 in the circumferential direction of the syringe 102 is restricted.

The inside of the syringe 102 is formed with a hollow portion 124 which can contain viscous material therein over the entire length in the longitudinal direction centering on the central axis C1. The hollow portion 124 in the tip portion 121 is formed into a tapered shape so that a cross-sectional size decreases toward the discharge opening 121a. Specifically, the hollow portion 124 in the tip portion 121 includes a first tapered portion 124a, a first columnar portion 124b, a second tapered portion 124c and a second columnar portion 124d. The first tapered portion 124a has one end continuous with the main body portion 123, and is configured so that a cross-sectional size decreases from the cross-sectional size in the main body portion 123 toward the discharge opening 121a. The first columnar portion 124b has one end continuous with the first tapered portion 124a, and is configured so that a cross-sectional size is constant, for example, an inner diameter of 0.1 mm to 13 mm. The second tapered portion 124c has one end continuous with the first columnar portion 124b, and is configured so that a cross-sectional size decreases from the cross-sectional size in the first columnar portion 124b toward the discharge opening 121a. The second columnar portion 124d has one end continuous with the second tapered portion 124c and the other end continuous with the discharge opening 121a, and is configured so that a cross-sectional size is constant, for example, an inner diameter of 0.1 mm to 10 mm.

In the main body portion 123, the hollow portion 124 is formed into a cylindrical shape so that a cross-sectional size is constant, for example, an inner diameter of 0.1 mm to 13 mm. In the rear end portion 122, the hollow portion 124 is formed into a cylindrical shape so that a cross-sectional size is constant, for example, an inner diameter of 0.1 mm to 15 mm. In particular, in the present embodiment, the hollow portion 121 in the rear end portion 122 is formed with a third tapered portion 124e so that a cross-sectional size slightly increases toward the insertion opening 122a in a portion continuous with the insertion opening 122a in order to facilitate the insertion of the push rod 104.

In the syringe 102 of the present embodiment, a syringe tip mounting portion 125 is formed on the outer peripheral side of the tip portion 121. In the present embodiment, the syringe tip mounting portion 125 is circularly provided around the central axis C1. In the present embodiment, the syringe tip mounting portion 125 is integrally formed with the syringe 102.

The syringe tip mounting portion 125 of the present embodiment has the same outer diameter as the main body 123, and the outer peripheral surface 125a of the syringe tip mounting portion 125 is configured to be substantially flush with the outer peripheral surface 123a of the main body portion 123. The syringe tip mounting portion 125 of the present embodiment is provided so that a gap is formed between the inner peripheral surface 125b of the syringe tip mounting portion 125 and the outer peripheral surface 121b of the tip portion 121. The inner peripheral surface 125b of the syringe tip mounting portion 125 of the present embodiment is provided with a internal thread. The syringe tip mounting portion 125 of the present embodiment is formed so that the diameter of the inner circumferential surface 125a slightly decreases toward a front end side in the longitudinal direction. The tip portion 121 is provided so as to extend toward the front end side in the longitudinal direction beyond the syringe tip mounting portion 125.

The tip portion 121 of the syringe 102 of the present embodiment has a shape that can abut with the cylindrical portion of the syringe tip 103 within the syringe tip 103 described below. Specifically, the end surface 121c on the front end side in the longitudinal direction of the tip portion 121 of the syringe 102 of the present embodiment is formed so as to be able to abut with the end surface on the rear end side in the longitudinal direction of the cylindrical portion of the syringe tip 103.

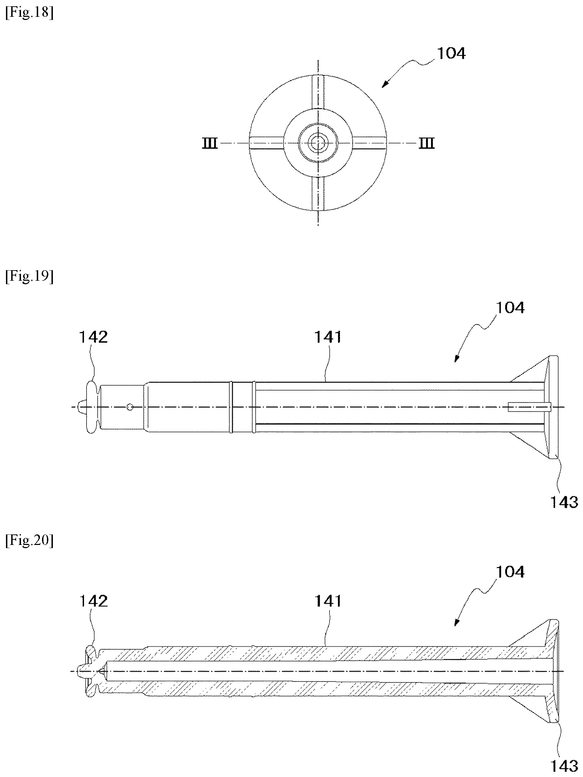

Figure 18 is a front view of the push rod 104 of the present embodiment. Figure 19 is a plan view of the push rod 104 of the present embodiment. Figure 20 is a sectional view of the push rod 104 along III-III line in Figure 18.

The push rod 104 is a member for pushing the viscous material contained in the syringe 102 toward the tip portion 121 (that is, the discharge opening 121a) of the syringe 102, and is made of, for example, a resin material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the push rod 104 is constructed from low density polyethylene.

The push rod 104 of the present embodiment has a main body portion 141 having a hollow cylindrical shape, a seal portion 142 formed at one end of the main body portion, and a finger hook portion 143 formed at the other end of the main body portion.

The main body portion 141 has a smaller outer diameter than the inner diameter of the syringe 102. Therefore, in the state that push rod 104 is contained in the syringe 102, there is a gap between the main body portion 141 and the syringe 102.

The seal portion 142 has a disc shape, and the outer peripheral end of the seal portion 142 is slidably and fluid-tightly in contact with the inner peripheral surface of the syringe 102. Although the seal portion 142 and the main body portion 141 are integrated as one member, the seal portion 142 and the main body portion 141 may be separate members.

Further, the seal portion 142 has an outer diameter larger than the inner diameter of the syringe 102 in order to fluid-tight contact the outer peripheral end with the inner peripheral surface of the syringe 102. The outer diameter of the seal portion 142 is larger than the outer diameter of the main body portion 141.

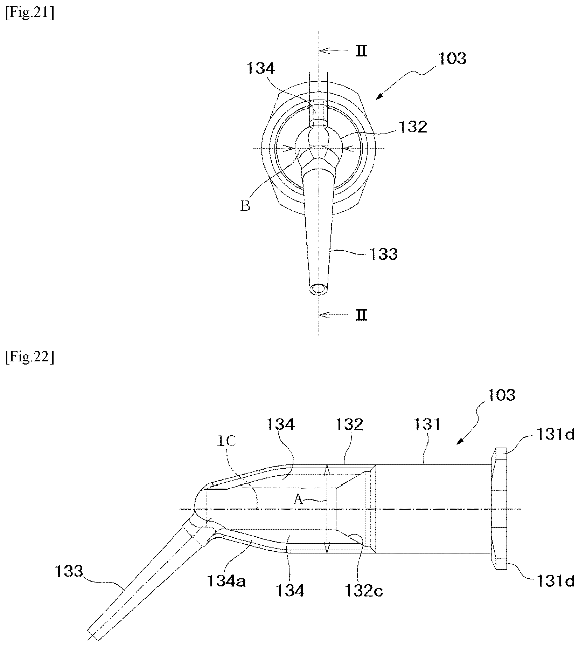

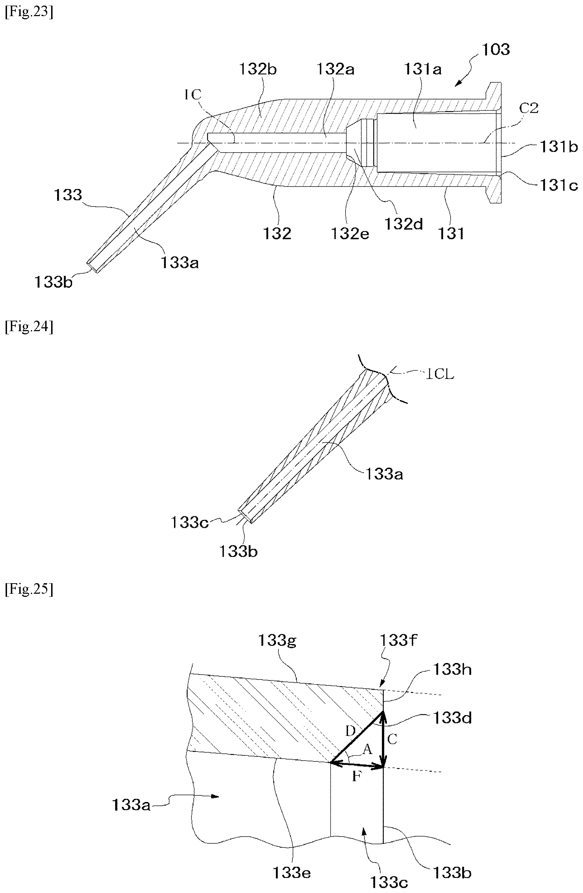

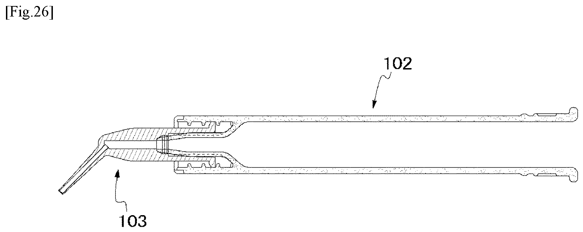

Figure 21 is a front view of a syringe tip according to one embodiment. Figure 22 is a plan view of a syringe tip according to one embodiment. Figure 23 is a sectional view along II-II line in Figure 21 Figure 24 is a Enlarged sectional view of the discharge opening portion of the syringe tip. Figure 25 is a schematic view of the discharge opening portion of the syringe tip. Figure 26 is a sectional view of syringe tip and syringe in the state of attaching the syringe tip to the tip of the syringe.

The syringe tip 103 of the present embodiment is attached to a tip of the syringe 102 containing viscous material to discharge the viscous material. The syringe tip 103 includes a syringe engaging portion 131 that engages with the syringe 102, a cylindrical portion 132 that has a hollow portion 132a having the center on the virtual central line IC and is held by the user when engaging the syringe tip 103 with the syringe 102, and a nozzle portion 133 that discharges the viscous material contained in the syringe 102.

The syringe engaging portion 131 of the present embodiment is a cylindrical member having the center on the central axis C2 and has a syringe insertion portion 131a in the inside of the syringe engaging portion 131. The tip portion of syringe 102 is inserted in the syringe insertion portion 131a. The outer diameter of the syringe engaging portion 131 may be, for example, 0.1 mm to 13 mm, and the dimension in the longitudinal direction of the syringe tip 103 may be, for example, 7 mm to 50 mm. In the present embodiment, the syringe insertion portion 131a is provided into a circular shape around the central axis C2, and may have an inner diameter of, for example, 0.1 mm to 12 mm. One end of the syringe insertion portion 131a constitutes an insertion opening 131b into which the syringe 102 is inserted. The other end of the syringe insertion portion 131a is continuous with the hollow portion 132a of the cylindrical portion 132.

In particular, the syringe insertion portion 131a of the present embodiment is formed into a tapered shape so that a cross-sectional size decreases toward the cylindrical portion 132. In the syringe insertion portion 131a of the present embodiment, in order to facilitate insertion of the syringe 102, a tapered portion 131c whose cross-sectional size slightly increases toward the insertion opening 131b is formed in a portion continuous with the insertion opening 131b.

An engagement piece 131d that is threadably attached with the internal thread of the syringe tip mounting portion 125 is projectaly mounted radially outward at the end on the insertion opening 131b side of the outer peripheral portion of the syringe engaging portion 131 of the present embodiment. In the present embodiment, a pair (two) of engagement pieces 131d are provided facing each other in the diametrical direction, and each engagement piece 131d is constituted by a flat projecting piece in the circumferential direction. In the present invention, three or more engagement pieces 131d may be provided, and it is also possible to provide a plurality of engagement pieces 131d with their positions shifted along the central axis C2. Further, each engagement piece 131d may be formed with a somewhat spiral slope.

The syringe engaging portion 131 may be formed of, for example, a resin material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the syringe engaging portion 131 is made of polypropylene. The material of the syringe engaging portion 131 may be the same as or different from the material of the syringe 102.

The cylindrical portion 132 of the present embodiment is a cylindrical member having the center on the virtual central line IC, and has a hollow portion 132a having the center on the virtual central line IC in the inside of the cylindrical portion 132. Specifically, the cylindrical portion 132 of the present embodiment has a main body portion 132b having a cylindrical shape and being formed with a hollow portion 132a in the inside of the main body portion 132b. The outer diameter of the cylindrical portion 132 may be, for example, 0.1 mm to 13 mm, and the dimension in the longitudinal direction of the syringe tip 103 may be, for example, 0.5 mm to 5 mm. One end of the hollow portion 132a of the present embodiment is connected to the syringe insertion portion 131a of the syringe engaging portion 131 and may have an inner diameter of, for example, 0.1 mm to 12 mm. The other end of the hollow portion 132a of the present embodiment is continuous with the flow path of the viscous material formed in the inside of the nozzle portion 133. The main body portion 132b of the present embodiment is formed so that a cross-sectional size is constant. Further, the hollow portion 132a of the present embodiment is formed so that a cross-sectional size is constant.

The cylindrical portion 132 may be formed of, for example, a resin material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the cylindrical portion 132 is made of polypropylene. The material of the cylindrical portion 132 may be the same as or different from the material of the syringe 102 and the syringe engaging portion 131.

In the present embodiment, the virtual central line IC positioned at the center of the hollow portion 132a is coaxial with the central axis C1 of the syringe 102 and the central axis C2 of the syringe insertion portion 131a.

The cylindrical portion 132 of the present embodiment has a connecting portion 132c that connects the main body portion 132b and the syringe engaging portion 131. The connecting portion 132c of the present embodiment is configured to have a truncated conical shape.

The connecting portion 132c of the present embodiment is formed with a hollow portion for connection 132d that connects the hollow portion 132a and the syringe insertion portion 131a of the syringe engaging portion 131 in the inside of the connecting portion 132c. The hollow portion for connection 132d of the present embodiment is configured to have a radial dimension having the center on the virtual central line IC (hereinafter simply referred to as radial dimension) that is larger than a radial dimension of the hollow portion 132a, and is smaller than a radial dimension of the syringe insertion portion 131a. The hollow portion for connection 132d of the present embodiment has, at the end on the hollow portion 132a side, an abutted surface 132e which has an annular shape and is abutted with the end surface 121c on the front end side in the longitudinal direction of the tip portion 121 of the syringe 102.

The cylindrical portion 132 of the present embodiment is provided with a pair of blade portions 134 on the outer wall portion of the main body portion 132b. The radial dimension of the blade portion 134 may be, for example, 8 mm to 12 mm, and the dimension in the longitudinal direction of the syringe tip 103 may be, for example, 0.5 mm to 5 mm. The pair of blade portions 134 of the present embodiment are provided so as to face each other in a radial direction having the center on the virtual central line IC. The pair of blade portions 134 of the present embodiment are formed from an end on the syringe engaging portion 131 side. In other words, the pair of blade portions 134 of the present embodiment are formed over between the main body portion 132b and the connecting portion 132c. In particular, the pair of blade portions 134 of the present embodiment are formed in a manner such that the outer end in the radial direction of the pair of blade portions 134 and the outer end in the radial direction of the syringe engaging portion 131 are flush with each other. Further, the pair of blade portions 134 of the present embodiment are configured to have a reduced diameter portion 134a on the nozzle portion 133 side. The radial dimension of reduced diameter portion 134a decreases toward the nozzle portion 133.

In the above embodiment, the cylindrical portion 132 includes the main body portion 132b formed with the hollow portion 132a, and the pair of blade portions 134 provided on the outer wall portion of the main body portion 132b.

The nozzle portion 133 discharges the viscous material contained in the syringe 102. Specifically, the nozzle portion 133 of the present embodiment is formed with a flow path 133a of viscous material in the inside of the nozzle portion 133, and one end of the flow path 133a is connected to a discharge opening 133b. A dimension of the nozzle portion 133 in the longitudinal direction of the syringe tip 103 may be, for example, 1 mm to 40 mm. Further, the dimension of the nozzle portion 133 in a direction in which the flow path 133a extends may be 5 mm or more, and may be 8 mm or more. By setting the dimension of the nozzle portion in a direction in which the flow path 133a extends to such a length, it is possible to easily fill the molars which are at the innermost part of the oral cavity with the viscous material, for example.

The nozzle portion 133 of the present embodiment is formed in a manner such that the cross-sectional size decreases toward the discharge opening 133b. However, the nozzle portion 133 may be formed in a manner such that the cross-sectional size is constant toward the discharge opening 133b and may be formed in a manner such that the cross-sectional size increases toward the discharge opening 133b. Further, the flow path 133a of the present embodiment is formed in a manner such that the cross-sectional size decreases toward the discharge opening 133b. Particularly, the flow path 133a of the present embodiment is configured to have a truncated conical shape. However, the flow path 133a may be formed in a manner such that the cross-sectional size is constant toward the discharge opening 133b and may be formed in a manner such that the cross-sectional size increases toward the discharge opening 133b. The flow path 133a may be a columnar shape.

The nozzle portion 133 may be formed of, for example, a resin material or a metal material. As the resin material, polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymerization) resin, styrene resin, polymethylpentene, methacrylstyrene, ABS (acrylonitrile, butadiene, styrene) resin, polycarbonate and the like can be used. In the present embodiment, the nozzle portion 133 is made of polypropylene.

In the syringe tip of the present embodiment, the nozzle portion, the cylindrical portion and the syringe engaging portion are integrally formed.

The nozzle portion 133 of the present embodiment extends in a direction inclining with respect to the virtual central line IC of the hollow portion 132a of the cylindrical portion 132. Further, the nozzle portion 133 is provided in a manner such that the extending direction of the nozzle portion 133 is parallel to the pair of blade portions.

As clearly shown in Figure 24, the flow path 133a of the present embodiment is formed around the virtual central line ICL. In the present specification, the virtual central line of the flow path may be defined as a line connecting the centers of gravity of the flow path in a cross section perpendicular to the moving direction of the viscous material. The nozzle portion 133 of the present embodiment has a tapered flow path 133c that is formed between the flow path 133a and the discharge opening 133b and the cross-sectional area of the tapered flow path 133c gradually increases toward the discharge opening 133b. In the present embodiment, the flow path 133a and the tapered flow path 133c are formed in the nozzle portion 133, and therefore, in the present embodiment, the discharge structure of the present invention is configured in the nozzle portion 133.

Since the nozzle portion 133 of the present embodiment comprises the discharge structure of the present invention, the cross-sectional area gradually increases toward the discharge opening 133b in the portion of the tapered flow path that is continuous with the discharge opening 133b. Therefore, it is possible to decrease the internal pressure of the viscous material flowing from the flow path 133a toward the discharge opening 133b in the tapered portion 133c, thereby reducing inherent strain. If the strain is present in the discharged viscous material, internal pressure is released during discharge, and deformation of the viscous material is caused. By reducing the strain, it is possible to reduce the deformation of the viscous material during discharge and to discharge the viscous material in the intended shape.

Further, in the nozzle portion 133 of the present embodiment, there is a tapered portion 133c that is continuous with the discharge opening 133b. Because the tapered portion 133c presents, the area of the circular tip surface 133h decreases, therefore the amount of viscous material that adheres to the circular tip surface 133h inevitably decrease. Thus, it is prevented that the inner surface of the cap from becoming dirty when the cap is fitted. In addition, the adhesion of the viscous material to undesired portion due to dripping of the paste adhering to the circular tip surface 133h is also suppressed. Furthermore, becaue the tapered portion 133c presents, it is possible that the viscous material discharged through the peripheral wall 133e, the peripheral wall 133d and the circular tip surface 133h pass smoothly without being caught at the edge of the discharge opening. Therefore, when the paste is pulled back by recovering the deformed push rod to the original shape in the case that the discharging of the viscous material is stopped, the viscous material is pulled back into the nozzle without remaining on the circular tip surface 133h. Thus, dripping of the viscous material can be reduced. Furthermore, the viscous material is smoothly pulled back into the nozzle, it is possible to improve the anti-dripping property of the viscous material.

Furthermore, in the nozzle portion 133 of the present embodiment, the cross-sectional area gradually increases toward the discharge opening 133b in the tapered portion 133c that is continuous with the discharge opening 133b, and therefore the internal volume of the nozzle portion increases. Thus, even if the pull back of the paste occurs by pull back of the push rod 104, because of increasing the paste capacity within the nozzle, it is possible that the air sucked from the tip portion remains in the nozzle tip to reduce the inclusion of air bubbles.

The tapered flow path 133c of the present embodiment has a truncated conical shape. Therefore, in the cross section including the virtual central line of the flow path 133a, the peripheral wall 133d of the tapered flow path 133c has a linear shape. In this way, by forming the peripheral wall 133d of the tapered flow path 133c into a linear shape in the cross section including the virtual central line of the flow path 133a, it is possible to improve the anti-dripping property of the viscous material.

In the nozzle portion 133 of the present embodiment, in the cross section of the flow path 133a including the virtual central line ICL of the flow path 133a, it is preferable that the angle between the extending direction of the peripheral wall 133e of the flow path 133a in the end portion on the discharge opening 133b side and the extending direction of the peripheral wall 133d of the tapered flow path 133c is 5° to 85°, more preferably 10° to 60°. By setting the angle to 5° to 85°, it is possible to prevent the viscous material from adhering to the outer wall portion 133f of the nozzle portion 133. In the present embodiment, the angle between the extending direction of the peripheral wall 133e of the flow path 133a in the end on the discharge opening 133b side and the extending direction of the peripheral wall 133d of the tapered flow path 133c is 45°.

In the nozzle portion 133 of the the present embodiment, the tapered flow path 133c and the outer peripheral wall portion 133g of the nozzle portion 133 are connected by a circular tip surface 133h. In particular, in the present embodiment, since the circular tip surface 133h has a flat annular shape, it is possible to improve the anti-dripping property of the viscous material.

In the present embodiment, in the case of viewing from the longitudinal direction of the syringe tip, the angle between the plane on which the pair of blade portions extend and the plane on which the nozzle portion extends may be 45° or less.

In the above embodiment, the tip portion of the syringe is configured to be able to abut with the syringe tip within the syringe tip. Therefore, it is possible to suppress the inclusion of air bubbles when the viscous material moves from the syringe to the syringe tip, and it is also possible to reduce the amount of viscous material remaining in the syringe tip and it is possible to suppress the viscous material from dripping from the syringe in the case of replacing the syringe tip.

In the syringe tip of the above embodiment, the nozzle portion, the cylindrical portion and the syringe engaging portion are integrally formed from a resin material. Therefore, press-fitting of the nozzle is not required during manufacturing, and it is possible to facilitate manufacturing. Further, since the nozzle portion, the cylindrical portion and the syringe engaging portion are made of only resin material, there is no need to separate metals at the time of disposal, and disposal can be easily performed.

In the above embodiment, a dental material is used as the viscous material, but pharmaceuticals, medical materials, cosmetics, foods and the like may be used as the viscous material.

In the above embodiment, the cylindrical portion comprises a main body portion formed with a hollow portion and a pair of blade portions provided on the outer wall portion of the main body portion. However, the syringe tip of the present invention may not have a blade portion. Further, in the above embodiment, two blade portions are provided, however the number of blade portion is not limited to two, and may be one, or three or more.

In the above embodiments, the syringe tip has a symmetrical shape, but the syringe tip may have an asymmetrical shape.

The syringe tip of the above embodiment is configured in a manner such that an extending direction of the nozzle portion is bent from an extending direction of the cylindrical portion, however, the nozzle portion and the cylindrical portion may be configured linearly.

In the syringe tip of the above embodiment, the flow path of the nozzle portion is formed linearly, however, the flow path of the nozzle portion may be formed in a curved shape.

In the syringe tip of the above embodiment, the nozzle portion, the cylindrical portion and the syringe engaging portion are integrally formed, however, the nozzle portion, the cylindrical portion and the syringe engaging portion may be individually formed as separate members.

In the syringe tip of the above embodiment, the nozzle portion 133 is formed in a manner such that the cross-sectional size decreases toward the discharge opening 133b, however, the cross-sectional size of the nozzle portion 133 may be constant.

In the syringe tip of the above embodiment, the flow path 133a is formed in a manner such that the cross-sectional size decreases toward the discharge opening 133b, however, the cross-sectional size of the flow path 133a may be constant.

In the above embodiment, the syringe tip and the syringe are threadably attached together using an internal thread type, but the syringe tip and the syringe may also be threadably attached together using a so-called external thread type.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### Industrial applicability

The present invention can provide a compule or syringe tip that can reduce an amount of viscous material adhering to an outer wall portion, preferably prevents adhesion of viscous material to an outer wall portion to apply a desired amount of viscous material to a desired portion to increase the efficiency of using viscous material.

### Reference Signs List

IC: virtual central line
1: compule
10: ejector holder
18: plunger
20: extending portion
22: handle member
24: actuation lever
30: button
31: engaging portion
31a: hollow portion
31b: insertion opening
32: cylindrical portion
32a: hollow portion
32b: main body portion
33: nozzle portion
33a: flow path
33b: discharge opening
33c: tapered flow path
33d: peripheral wall
33g: outer peripheral wall portion
33h: tip surface
34: piston
35: flange
101: injector
102: syringe
121: tip portion
121a: discharge opening
121b: outer peripheral surface
121c: end surface
122: rear end portion
122a: insertion opening
122b: finger grip attachment portion
122c: flange portion
122d: flange portion
122e: recess portion
123: main body portion
123a: outer peripheral surface
124: hollow portion
124a: first tapered portion
124b: first columnar portion
124c: second tapered portion
124d: second columnar portion
124e: third tapered portion
125: syringe tip mounting portion
125a: outer peripheral surface
125b: inner peripheral surface
103: syringe tip
131: syringe engaging portion
131a: syringe insertion portion
131b insertion opening
131c: tapered portion
131d engagement piece
132: cylindrical portion
132a: hollow portion
132b: main body portion
132c: connecting portion
132d: hollow portion for connection
132e: abutted surface
133: nozzle portion
133a: flow path
133b: discharge opening
133c: tapered flow path
133d: peripheral wall
133e: peripheral wall
133f: outer wall portion
133g: outer peripheral wall portion
133h: tip surface
134: blade portion
134a: reduced diameter portion
104: push rod
141: main body portion
142: seal portion
143: finger hook portion
105: finger grip
C1: central axis
C2: central axis
IC: virtual central line

## Claims

1. A compule or syringe tip having a discharge structure for discharging viscous material from a discharge opening, wherein
the discharge structure comprises
a flow path that is formed around a virtual central line and allows the viscous material to move, and
a tapered flow path formed between the flow path and the discharge opening, wherein
a cross-sectional area of the tapered flow path gradually increases toward the discharge opening.

2. The compule or syringe tip according to claim 1, wherein
in the cross section of the flow path including the virtual center line, an angle between an extending direction of a peripheral wall of the flow path in the end portion on a discharge opening side and an extending direction of a peripheral wall of the tapered flow path is 5° to 85°.

3. The compule or syringe tip according to claim 1 or 2, wherein
the tapered flow path has a truncated conical shape.

4. The compule or syringe tip according to any one of claims 1 to 3, wherein
the flow path has a truncated conical shape or a columnar shape.

5. The compule or syringe tip according to any one of claims 1 to 4, wherein
the tapered flow path and an outer peripheral wall portion of a discharge container or an outer peripheral wall portion of a discharge instrument are connected by a circular tip surface.

6. The compule or syringe tip according to claim 5, wherein
the circular tip surface has an annular shape.

7. The compule according to any one of claims 1 to 6.

8. The syringe tip according to any one of claims 1 to 6.
